# EUROPEAN PATENT SPECIFICATION

(11) **EP 2 285 367 B1**
(45) Date of publication and mention of the grant of the patent: **22.02.2012**
(21) Application number: 09745819.4
(22) Date of filing: 15.05.2009
(51) Int. Cl.: A61K 31/17, A61K 31/80, A61K 36/185, A61K 36/61, A61K 36/736, A61K 36/67, A61K 36/752, A61K 36/54, A61K 36/53, A61K 36/14, A61P 17/12

(54) **PHARMACEUTICAL COMPOSITIONS FOR THE TREATMENT OF WARTS**
PHARMAZEUTISCHE ZUSAMMENSETZUNGEN ZUR BEHANDLUNG VON WARZEN
COMPOSITIONS PHARMACEUTIQUES DESTINÉES AU TRAITEMENT DES VERRUES

(30) Priority: 15.05.2008 NL 1035426
(43) Date of publication of application: 23.02.2011
(73) Proprietor: ATP Marketing & Promotion AG, 8867 Niederurnen (CH)
(72) Inventor: OLDANI, Mario, Dominik, Nicolaas, NL-4233 CH Ameide (NL)
(74) Representative: van Kooij, Adriaan
(86) International application number: PCT/EP2009/055904
(87) International publication number: WO 2009/138487

(56) References cited:
- US-A1- 2002 197 291
- US-A1- 2007 196 452
- HIGASHI N ET AL: "Recalcitrant plantar warts treated with topical 10% urea ointment" SKIN RESEARCH, vol. 42, no. 5, 2000, pages 498-502, XP008099574
- HIGASHI NOBUHIKO ET AL: "Treatment of Verruca Plana with Urea Ointment (Urepal)" SKIN RESEARCH, vol. 36, no. 1, 1994, pages 55-59, XP008099573

## Description

The present invention relates to pharmaceutical compositions for use in the treatment, and especially the topical treatment, of warts and the use thereof for the treatment of warts.

A wart (Verrucca), is a hard, or horny, callous deformity of the upper skin generally in the form of a cauliflower-like nodule. At least four different types of warts are known: the common wart (*Verruca vulgaris*), the plantar wart (*verucca plantaris*), the flat wart (*Verucca plana*) and the filiform wart (*Verucca filiformis*).

Warts generally comprise a core of connective tissue, and comprised therein blood vessels, coated with one or more layers of epithelial tissue. Warts can occur alone or in groups.

Warts are generally caused by human papilloma viruses. Papilloma virus infected skin cells grow faster than non-infected skin cells. This growth difference, in comparison with the surrounding skin cells, results in a wart. Because of their virulent nature, warts are contagious.

Although in most cases warts do not cause other adverse medical conditions, and even spontaneously disappear after a certain time period, individuals receiving organ transplants, or other individuals with a compromised immune system, often develop warts on their hands and lower arms which warts sometimes appear to develop into squamous cell carcinomas.

For the treatment of warts, several methods are known from the prior art.
1) Application of podophyllum resin paint [podophyllum resin I.P.'66 (20% w/v), benzoin I.P. (10% w/v), aloes I.P. (2% w/v), isopropyl alcohol I.P. to make (100% v/v)];
2) Keratolysis, the removal of dead surface skin cells usually using salicylic acid, blistering agents, immune system modifiers ("immunomodulators"), or formaldehyde, often with mechanical paring of the wart with a pumice stone, blade etc.;
3) Cryosurgery, which involves freezing the wart (generally with liquid nitrogen), creating a blister between the wart and epidermal layer, after which the wart and surrounding dead skin falls off. An average of 3 to 4 treatment is required for warts of thin skin. Warts on calloused skin like plantar warts might take dozens or more treatments.
4) Surgical curettage of the wart;
5) Laser treatment, often with a pulse dye laser or carbon dioxide (CO₂) laser. Pulse dye lasers work by selective absorption by blood cells (specifically haemoglobin). CO₂ lasers work by selective absorption by water molecules. Pulse dye lasers are less destructive and more likely to heal without scarring. CO₂ laser works by vaporizing and destroying tissue and skin. Both laser treatments can be painful, expensive, and can cause scarring. CO₂ lasers will require local anaesthetic, while pulse dye lasers might need conscious sedation. The therapy comprises 1 to 4 treatments.
6) Infrared coagulator, an intense source of infrared light in a small beam like a laser. This works essentially on the same principle as laser treatment. Like the laser, it can cause blistering, pain and scarring.
7) Imiquimod, a topical cream that helps the body's immune system combat the wart virus by encouraging interferon production.
8) Injection of *Candida,* mumps, or *Trichophyton* antigens at the site of the wart, which stimulates the body's immune system.
9) Cantharidin, a chemical found naturally in many members of the beetle family *Meloidae* which causes dermal blistering. Either used by itself or compounded with podophyllin.
10) Bleomycin, one or two injections are used. Bleomycin can cause necrosis of digits and Raynaud syndrome.
11) Dinitrochlorobenzene (DNCB), like salicylic acid, this is applied directly onto the wart. Studies showed this method was effective with a cure rate of 80% compared to 38% for a placebo. But DNCB must be used much more cautiously than salicylic acid; the chemical is a known mutagen and, therefore, capable of causing genetic mutations. This drug induces an allergic immune response resulting in inflammation that wards off the wart-causing virus.
12) Fluorouracil, which inhibits DNA synthesis, is being used as an experimental treatment. It is applied directly to the wart (especially plantar warts) and covered with, for example, tape. This treatment is combined with the use of a pumice stone, but tends to work very slowly.
However, none of the above mentioned methods provide an effective, or satisfying treatment of warts.

Accordingly, it an object of the present invention to provide novel pharmaceutical compositions for the treatment, and especially the topical treatment, of warts.

This object, amongst other objects, is met by the pharmaceutical compositions for the treatment of warts as defined in the appended claim 1.

Specifically, this object, amongst other objects, is met by pharmaceutical compositions for the treatment of warts comprising:
a) 0.1 to 10 weight% dimethicone;
b) 5 to 25 weight% of one or more vegetable oils;
c) one or more pharmaceutically acceptable excipients and/or carriers;
d) 2 to 6 weight% urea; and
e) water up to 100 weight%.

As used herein, the term weight% relates to the weight of the indicated ingredient divided by the weight of the total pharmaceutical composition and multiplied by 100%.

Dimethicone (polydimethylsolixane, PDMS) is a flexible silicon polymer generally used in hand lotions and cosmetic products. The present inventor surprisingly discovered that besides this known use of dimethicone, dimethicone also has a beneficial effect on the treatment of warts.

The pharmaceutical compositions according to the present invention comprise urea. Urea, because of its water retention (wetting) characteristics is one of the most important water retention compounds of the skin.

Besides dimethicone and urea, the present pharmaceutical compositions also beneficially comprise one or more vegetable oils and one or more pharmaceutically acceptable excipients and/or carriers.

In a preferred embodiment of the present invention, the one or more vegetable oils are chosen from the group consisting of almond oil, tea tree oil, lavender oil, geranium oil, lemon oil, thuja oil, and cinnamon oil.

Almond oil, for example, beneficially regulates the moisture content of the skin and reduces skin irritations.

Lavender oil, for example, beneficially provides disinfecting, bactericide, pain killing, curing and purifying characteristics.

Geranium oil, for example, beneficially provides infection inhibiting, fungus inhibiting and wound healing characteristics.

Tea tree oil, for example, beneficially provides bacterial, viral and fungal inhibiting growth characteristics. Further, tea tree oil is pain and scar reducing, and improves healing. Furthermore, tea tree oil readily mixes with natural skin oils allowing a quick effect on the source of infection.

Cinnamon oil, for example, aids in combating bacteria, viruses and fungi, is inflammation inhibiting and improves the blood flow.

Thuja oil, for example, is a bactericide and fungicide.

Lemon oil, for example, is disinfecting.

According to a preferred embodiment, the present pharmaceutical composition further comprises 0.5 to 2 weight% red pepper powder. Red pepper, also designated as Chilean or Spanish pepper or hot pepper, is rich in vitamin C, reduces pain, stimulates nerves, and is disinfecting.

Preferably, the present one or more excipients and/or carriers are chosen from the group consisting of ammonium acryloyldimethyl taurate/ VP copolymer, sodium benzoate, potassium sorbate and citric acid.

According to an especially preferred embodiment of the present invention, the pharmaceutical compositions comprise:
a) 0.5 to 3 weight% dimethicone;
b) vegetable oils comprising:
   1) 1 to 3 weight% almond oil;
   2) 1 to 3 weight% tea tree oil;
   3) 1 to 2 weight% lavender oil;
   4) 1 to 2 weight% geranium oil;
   5) 0.5 to 2 weight% lemon oil;
   6) 0.5 to 2 weight% thuja oil;
   7) 0.5 to 2 weight% cinnamon oil.
c) pharmaceutically acceptable excipients and/or carriers comprising:
   1) 0.5 to 3 weight% ammonium acryloyldimethyl taurate/ VP copolymer;
   2) 0.1 to 0.5 weight% sodium benzoate;
   3) 0.05 to 0.25 weight% potassium sorbate;
   4) 0.05 to 0.25 weight% citric acid;
d) 2 to 6 weight% urea;
e) 0.5 to 3 weight% red pepper powder;
f) water up to 100 weight%.

According to a most preferred embodiment of the present invention, the pharmaceutical compositions comprise:
a) 1 weight% dimethicone;
b) vegetable oils comprising:
   1) 2 weight% almond oil;
   2) 2 weight% tea tree oil;
   3) 1.5 weight% lavender oil;
   4) 1.5 weight% geranium oil;
   5) 1 weight% lemon oil;
   6) 1 weight% thuja oil;
   7) 1 weight% cinnamon oil.
c) pharmaceutically acceptable excipients and/or carriers comprising:
   1) 1.65 weight% ammonium acryloyldimethyl taurate/ VP copolymer;
   2) 0.3 weight% sodium benzoate;
   3) 0.16 weight% potassium sorbate;
   4) 0.15 weight% citric acid;
d) 4 weight% urea;
e) 1 weight% red pepper powder;
f) water up to 100 weight%.

The pharmaceutical compositions according to the present invention are, in a preferred embodiment, formulated for topical administration, preferably in the form of an ointment, crème or tincture.

The present pharmaceutical compositions are especially effective for the treatment of warts. Accordingly, the present invention also relates to dimethicone for the topical treatment of warts and the use of dimethicone for the preparation of a medicament for the topical treatment of warts.

A beneficial effect of the present pharmaceutical compositions, and the use thereof, is further provided by the observation that the wart surrounding skin substantially remains unaffected by the present compositions.

The present pharmaceutical compositions are suitably, for example and preferably, topically applied, 1 to 5 times a day, preferably 1 to 2 times a day, on the wart. After application, the present pharmaceutical composition is massaged in and allowed to be absorbed. The indicated treatment is repeated until the wart has at least visibly disappeared.

The indicated treatment regime using the present compositions has been used by a number of individuals repeatedly suffering from warts and a significant subset of these individuals reported the disappearance of the warts within a time period of 1 week to several months without pain, scarring or any other side effects.

## Claims

1. Pharmaceutical composition for use in the treatment of warts, comprising:
a) 0.1 to 10 weight% dimethicone;
b) 5 to 25 weight% of one or more vegetable oils;
c) one or more pharmaceutically acceptable excipients and/or carriers;
d) 2 to 6 weight% urea; and
e) water up to 100 weight%.

2. Pharmaceutical composition for use according to claim 1, wherein said one or more vegetable oils are chosen from the group consisting of almond oil, tea tree oil, lavender oil, geranium oil, lemon oil, thuja oil, and cinnamon oil.

3. Pharmaceutical composition for use according to claim 1 or claim 2, further comprising:
f) 0.5 to 2 weight% red pepper powder.

4. Pharmaceutical composition for use according to any of the claims 1 to 3, wherein said one or more pharmaceutically acceptable excipients and/or carriers are chosen from the group consisting of ammonium acryloyldimethyl taurate/ VP copolymer, sodium benzoate, potassium sorbate and citric acid.

5. Pharmaceutical composition for use according to any of the claims 1 to 4, comprising:
a) 0.5 to 3 weight% dimethicone;
b) vegetable oils comprising:
1) 1 to 3 weight% almond oil;
2) 1 to 3 weight% tea tree oil;
3) 1 to 2 weight% lavender oil;
4) 1 to 2 weight% geranium oil;
5) 0.5 to 2 weight% lemon oil;
6) 0.5 to 2 weight% thuja oil;
7) 0.5 to 2 weight% cinnamon oil.
c) pharmaceutically acceptable excipients and/or carriers comprising:
1) 0.5 to 3 weight% ammonium acryloyldimethyl taurate/ VP copolymer;
2) 0.1 to 0.5 weight% sodium benzoate;
3) 0.05 to 0.25 weight% potassium sorbate;
4) 0.05 to 0.25 weight% citric acid;
d) 2 to 6 weight% urea;
e) 0.5 to 3 weight% red pepper powder;
f) water up to 100 weight%.

6. Pharmaceutical composition for use according to any one of the claims 1 to 5, comprising:
a) 1 weight% dimethicone;
b) vegetable oils comprising:
1) 2 weight% almond oil;
2) 2 weight% tea tree oil;
3) 1.5 weight% lavender oil;
4) 1.5 weight% geranium oil;
5) 1 weight% lemon oil;
6) 1 weight% thuja oil;
7) 1 weight% cinnamon oil.
c) pharmaceutically acceptable excipients and/or carriers comprising:
1) 1.65 weight% ammonium acryloyldimethyl taurate/ VP copolymer;
2) 0.3 weight% sodium benzoate;
3) 0.16 weight% potassium sorbate;
4) 0.15 weight% citric acid;
d) 4 weight% urea;
e) 1 weight% red pepper powder;
f) water up to 100 weight%.

7. Pharmaceutical composition for use according to any of the claims 1 to 6 in a dosage form for topical administration.

8. Pharmaceutical composition for use according to claim 7, wherein the topical dosage form is an ointment, creme or tincture.

9. Dimethicone for use in the topical treatment of warts.

10. Use of dimethicone for the preparation of a medicament for the topical treatment of warts.

## Patentansprüche

1. Pharmazeutische Zusammensetzung zur Verwendung bei der Behandlung von Warzen, umfassend:
a) 0,1 bis 10 Gew.-% Dimethicon,
b) 5 bis 25 Gew.-% von einem oder mehreren pflanzlichen Ölen,
c) einen oder mehrere pharmazeutisch annehmbare Hilfsstoffe und / oder Träger,
d) 2 bis 6 Gew.-% Harnstoff, und
e) Wasser bis auf 100 Gew.-%.

2. Pharmazeutische Zusammensetzung zur Verwendung gemäß Anspruch 1, wobei das eine oder die mehreren pflanzlichen Öle aus der Gruppe bestehend aus Mandelöl, Teebaumöl, Lavendelöl, Geranienöl, Zitronenöl, Thujaöl und Zimtöl ausgewählt wird / werden.

3. Pharmazeutische Zusammensetzung zur Verwendung gemäß Anspruch 1 oder 2, ferner umfassend:
f) 0,5 bis 2 Gew.-% Cayennepfefferpulver.

4. Pharmazeutische Zusammensetzung zur Verwendung gemäß einem der Ansprüche 1 bis 3, wobei der eine oder die mehreren pharmazeutisch annehmbaren Hilfsstoffe und / oder Träger aus der Gruppe bestehend aus Ammoniumacryloyldimethyltaurat / VP-Copolymer, Natriumbenzoat, Kaliumsorbat und Zitronensäure ausgewählt wird / werden.

5. Pharmazeutische Zusammensetzung zur Verwendung gemäß einem der Ansprüche 1 bis 4, umfassend:
a) 0,5 bis 3 Gew.-% Dimethicon,
b) pflanzliche Öle, umfassend:
1) 1 bis 3 Gew.-% Mandelöl,
2) 1 bis 3 Gew.-% Teebaumöl,
3) 1 bis 2 Gew.-% Lavendelöl,
4) 1 bis 2 Gew.-% Geranienöl,
5) 0,5 bis 2 Gew.-% Zitronenöl,
6) 0,5 bis 2 Gew.-% Thujaöl,
7) 0,5 bis 2 Gew.-% Zimtöl,
c) pharmazeutisch annehmbare Hilfsstoffe und / oder Träger, umfassend:
1) 0,5 bis 3 Gew.-% Ammoniumacryloyldimethyltaurat / VP-Copolymer,
2) 0,1 bis 0,5 Gew.-% Natriumbenzoat,
3) 0,05 bis 0,25 Gew.-% Kaliumsorbat,
4) 0,05 bis 0,25 Gew.-% Zitronensäure,
d) 2 bis 6 Gew.-% Harnstoff,
e) 0,5 bis 3 Gew.-% Cayennepfefferpulver,
f) Wasser bis auf 100 Gew.-%.

6. Pharmazeutische Zusammensetzung zur Verwendung gemäß einem der Ansprüche 1 bis 5, umfassend:
a) 1 Gew-% Dimethicon,
b) pflanzliche Öle, umfassend:
1) 2 Gew.-% Mandelöl,
2) 2 Gew.-% Teebaumöl,
3) 1,5 Gew.-% Lavendelöl,
4) 1,5 Gew.-% Geranienöl,
5) 1 Gew.-% Zitronenöl,
6) 1 Gew.-% Thujaöl,
7) 1 Gew.-% Zimtöl,
c) pharmazeutisch annehmbare Arzneimittelträger und / oder Träger, umfassend:
1) 1,65 Gew.-% Ammoniumacryloyldimethyltaurat / VP-Copolymer,
2) 0,3 Gew.-% Natriumbenzoat,
3) 0,16 Gew.-% Kaliumsorbat,
4) 0,15 Gew.-% Zitronensäure,
d) 4 Gew.-% Harnstoff,
e) 1 Gew.-% Cayennepfefferpulver,
f) Wasser bis auf 100 Gew.-%.

7. Pharmazeutische Zusammensetzung zur Verwendung gemäß einem der Ansprüche 1 bis 6 in einer Dosierungsform zur topischen Verabreichung.

8. Pharmazeutische Zusammensetzung zur Verwendung gemäß Anspruch 7, wobei die topische Dosierungsform eine Salbe, eine Creme oder eine Tinktur ist.

9. Dimethicon zur Verwendung bei der topischen Behandlung von Warzen.

10. Verwendung von Dimethicon für die Herstellung eines Medikaments für die topische Behandlung von Warzen.

## Revendications

1. Composition pharmaceutique destinée à une utilisation pour le traitement des verrues, comprenant:
a) 0,1 % à 10 % en poids de diméthicone;
b) 5 % à 25 % en poids d'une ou plusieurs huiles végétales;
c) un ou plusieurs excipients et/ou supports pharmaceutiquement acceptables;
d) 2 % à 6 % en poids d'urée; et
e) de l'eau jusqu'à 100 % en poids.

2. Composition pharmaceutique destinée à une utilisation selon la revendication 1, où lesdites une ou plusieurs huiles végétales sont choisies parmi le groupe consistant en huile d'amande, huile d'arbre à thé, huile de lavande, huile de géranium, huile de citron, huile de thuya, et huile de cannelle.

3. Composition pharmaceutique destinée à une utilisation selon la revendication 1 ou la revendication 2, comprenant en outre:
f) 0,5 % à 2 % en poids de poudre de poivre rouge.

4. Composition pharmaceutique destinée à une utilisation selon l'une quelconque des revendications 1 à 3, où lesdits un ou plusieurs excipients et/ou supports pharmaceutiquement acceptables sont choisis par le groupe consistant en copolymère d'acryloyldiméthyl taurate d'ammonium/VP, benzoate de sodium, sorbate de potassium et acide citrique.

5. Composition pharmaceutique destinée à une utilisation selon l'une quelconque des revendications 1 à 4, comprenant:
a) 0,5 % à 3 % en poids de diméthicone;
b) des huiles végétales comprenant:
1) 1 % à 3 % en poids d'huile d'amande;
2) 1 % à 3 % en poids d'huile d'arbre à thé;
3) 1 % à 2 % en poids d'huile de lavande;
4) 1 % à 2 % en poids d'huile de géranium;
5) 0,5 % à 2 % en poids d'huile de citron;
6) 0,5 % à 2 % en poids d'huile de thuya;
7) 0,5 % à 2 % en poids d'huile de cannelle;
c) des excipients et/ou supports pharmaceutiquement acceptables comprenant:
1) 0,5 % à 3 % en poids d'un copolymère d'acryloyldiméthyl taurate d'ammonium/VP;
2) 0,1 % à 0,5 % en poids de benzoate de sodium;
3) 0,05 % à 0,25 % en poids de sorbate de potassium;
4) 0,05 ô à 0,25 % en poids d'acide citrique;
d) 2 % à 6 % en poids d'urée;
e) 0,5 % à 3 % en poids de poudre de poivre rouge;
f) de l'eau jusqu'à 100 % en poids.

6. Composition pharmaceutique destinée à une utilisation selon l'une quelconque des revendications 1 à 5, comprenant:
a) 1 % en poids de diméthicone;
b) des huiles végétales comprenant:
1) 2 % en poids d'huile d'amande;
2) 2 % en poids d'huile d'arbre à thé;
3) 1,5 % en poids d'huile de lavande;
4) 1,5 % en poids d'huile de géranium;
5) 1 % en poids d'huile de citron;
6) 1 % en poids d'huile de thuya;
7) 1 % en poids d'huile de cannelle;
c) des excipients et/ou supports pharmaceutiquement acceptables comprenant:
1) 1,65 % en poids d'un copolymère d'acryloyldiméthyl taurate d'ammonium/VP;
2) 0,3 % en poids de benzoate de sodium;
3) 0,16 % en poids de sorbate de potassium;
4) 0,15 % en poids d'acide citrique;
d) 4 % en poids d'urée;
e) 1 % en poids de poudre de poivre rouge;
f) de l'eau jusqu'à 100 % en poids.

7. Composition pharmaceutique destinée à une utilisation selon l'une quelconque des revendications 1 à 6 sous une forme pharmaceutique pour administration topique.

8. Composition pharmaceutique destinée à une utilisation selon la revendication 7, où la forme pharmaceutique topique est un onguent, une crème ou une teinture.

9. Diméthicone destinée à une utilisation dans le traitement topique des verrues.

10. Utilisation de diméthicone pour la préparation d'un médicament destiné au traitement topique des verrues.
